Europäisches Patentamt

European Patent Office

Office européen des brevets

(11) Veröffentlichungsnummer: **0 048 382**
**B1**

## (12) EUROPÄISCHE PATENTSCHRIFT

(45) Veröffentlichungstag der Patentschrift:
27.12.84

(51) Int. Cl.³: **G 03 B 41/16, A 61 B 6/00**

(21) Anmeldenummer: **81107038.2**

(22) Anmeldetag: **07.09.81**

(54) Röntgenaufnahmeeinrichtung mit einem von einer Bereitschaftsstellung in eine Aufnahmestellung verfahrbaren Filmträger.

(30) Priorität: **19.09.80 DE 3035448**

(43) Veröffentlichungstag der Anmeldung:
**31.03.82 Patentblatt 82/13**

(45) Bekanntmachung des Hinweises auf die Patenterteilung:
**27.12.84 Patentblatt 84/52**

(84) Benannte Vertragsstaaten:
**BE FR IT**

(56) Entgegenhaltungen:
**DE - A - 2 744 139**
**DE - B - 2 415 410**
**DE - C - 2 044 848**
**US - A - 3 502 878**
**US - A - 3 920 997**

(73) Patentinhaber: **Siemens Aktiengesellschaft, Berlin und München Wittelsbacherplatz 2, D-8000 München 2 (DE)**

(72) Erfinder: **Adelmeyer, Dieter, Erlenweg 21, D-8551 Adelsdorf/Aisch (DE)**
Erfinder: **Duschka, Hartmut, Amselweg 5, D-8521 Uttenreuth (DE)**
Erfinder: **Schott, Helmut, Paul-Gerhardt-Strasse 13, D-8510 Fürth (DE)**

## Beschreibung

Die Erfindung bezieht sich auf eine Röntgenaufnahmeeinrichtung mit einem in Führungen von einer Bereitschaftsstellung in mindestens eine Aufnahmestellung und wieder zurück verfahrbaren Filmträger mit verschiebbaren Spannbacken für die Halterung von Röntgenfilmkassetten unterschiedlichen Formates.

Durch die DE-B-2 044 848 ist ein Röntgenzielgerät oder Röntgenkassettenwechsler bekannt, bei dem eine Röntgenfilmkassette motorisch von einer Bereitschaftsstellung in eine Aufnahmestellung verfahrbar ist. Bei diesem Röntgenzielgerät bzw. Röntgenkassettenwechsler werden von den an den Kassettenkanten zur Anlage bringbaren Spannbacken Spannungsteiler verstellt. Deren Widerstandswerte werden zusammen mit einem konstanten Wert für die Wegvorgabe zur Steuerung des Einfahrweges des Kassettenwagens von der Bereitschaftsstellung in die Aufnahmestellung einer motorischen Nachlaufsteuerung zugeschaltet. Diese Widerstandswerte können auch einer weiteren Nachlaufsteuerung zur Voreinstellung der Primärstrahlenblende zugeschaltet werden. Es ist eine Eigenart dieser Konstruktion, dass die Spannungsteiler, die die abgegriffenen Kassettenmasse in elektrische Grössen umwandeln, in einer elektrischen Verbindung mit der Röntgenaufnahmeeinrichtung und im Falle einer entsprechenden Steuerung der Primärstrahlenblende auch in einer elektrischen Verbindung mit dem übrigen Röntgenuntersuchungsgerät stehen müssen. Dies hat zur Folge, dass die an den Spannbacken angekuppelten Spannungsteiler entweder fest mit dem übrigen Röntgenuntersuchungsgerät verdrahtet sein müssen oder aber an diesem mittels einer Steckverbindung anzuschliessen sind. Beides verhindert oder behindert die Entnahme des Filmträgers.

In der US-A-3 502 878 ist eine Röntgenaufnahmeeinrichtung beschrieben, bei der eine Primärstrahlenblende die Grösse des Röntgenstrahlenbündels in Abhängigkeit von der Kassettengrösse und den Film-Fokus-Abstand derart begrenzt, dass die zu belichtende Fläche des Röntgenfilmes und die Fläche der Projektion des Röntgenstrahlenbündels auf der Filmebene die gleiche Grösse aufweisen. Es sind zwei speziell geformte Spannbacken für die Kassette vorgesehen, deren Randbereiche treppenförmig ausgebildet sind, so dass durch die Aussparungen die Ecken von Kassetten verschiedener Standardgrössen erfasst werden können. Die Auflageflächen der Aussparungen sind mit Kontakten versehen, die die Kassettenbreite erfassen und als Widerstandswert einem Servoverstärker zuführen. Der Abstand der Spannbacken, der die Kassettenlänge repräsentiert, wird ebenfalls über Kontakte ermittelt und einem weiteren Servoverstärker zugeführt. Die Servoverstärker verstellen in Abhängigkeit von den ihnen zugeführten Widerstandswerten die Blendenöffnung der Primärstrahlenblende. Durch die spezielle Ausführungsform der Spannbacken lassen sich nur bestimmte standarisierte Kassettengrössen verwenden. Auch stehen zumindest die Kontakte in elektrischer Verbindung mit der Röntgenaufnahmeeinrichtung, so dass die Entnahme des Filmträgers verhindert oder bei Steckverbindungen behindert wird.

Die DE-C-2 415 410 offenbart ein unter einem Untersuchungstisch einschiebbares Kassettenblech, bei dem die Spannbacken bei der Anlage an den Kassettenkanten einen über einen Seilzug im Kassettenblech quer zur Einschubrichtung verschiebbar gelagerten Permanentmagneten verstellen. Dieser Permanentmagnet aktiviert je nach Stellung bei eingeschobenem Kassettenblech einen von mehreren, im Längslaufwagen des Untersuchungstisches eingebauten Reed-Kontakt. Dieser Reed-Kontakt schaltet dann einen entsprechenden Widerstand in eine Nachlaufsteuerung für die Primärstrahlenblende und stellt diese so auf die abgegriffenen Kassettenmasse ein. Es ist eine Eigenart dieser Konstruktion, dass das Kassettenblech zwar ohne elektrische Anschlüsse auskommt und daher ohne weiteres aus dem Untersuchungstisch entfernt werden kann, dafür aber nur bei bestimmten diskreten angepassten Kassettenabmessungen benutzt werden kann.

Um die Primärstrahlenblende auch auf Röntgenfilmkassetten beliebigen Formates einstellen zu können, ist es durch die DE-A1-2 744 139 bekanntgeworden, an dem im Untersuchungstisch verbleibenden rahmenförmigen Längslaufwagen Fühler anzubringen, die sich beim Einschalten einer auf einem Kassettenblech eingespannten Röntgenfilmkassette aussen an die Spannbacken des Kassettenbleches anlegen und über ein Getriebe je ein separates Potentiometer für die Breite und für die Höhe der Röntgenfilmkassette verstellen. Solche Fühler müssen relativ schmal und lang sein und haben daher die Eigenschaft, sich leicht zu verbiegen. Dabei wird entweder die Einschuböffnung des Kassettenbleches blockiert oder es kommt infolge Vorbeigleitens an den Spannbacken des Kassettenbleches zu Fehlbelichtungen. Ausserdem ist ein erheblicher feinmechanischer Aufwand für die Übertragung der Bewegung der Fühler auf entsprechende Potentiometer erforderlich. Bereits geringe Winkeländerungen können zu deutlich messbaren Widerstandsänderungen führen.

Der vorliegenden Erfindung liegt daher die Aufgabe zugrunde, einen Weg zu weisen, wie die Vorzüge einer automatischen Strahleneinblendung und einer automatischen Wegvorgabe aufgrund der abgegriffenen Kassettenabmessungen auch für Röntgenzielgeräte, Flachblenden und solche Kassettenbleche nutzbar zu machen sind, die bei solchen Untersuchungen, bei denen sie nicht benötigt werden, wieder vollständig aus der Röntgenaufnahmeeinrichtung entfernt werden können, um die volle Bewegungsfreiheit der übrigen Bausteine des Röntgenuntersuchungsgerätes nicht zu beeinträchtigen. Darüber hinaus sollte diese Lösung die Verwendung von Kassetten beliebiger Grösse ermöglichen und zugleich robust und preiswert sein.

Bei einer Röntgenaufnahmeeinrichtung der eingangs genannten Art sind daher erfindungsgemäss der Filmträger und zumindest ein Teil der Spannbacken mit Gebern gekuppelt, wobei die Geber der Spannbacken in Abhängigkeit von der Spannweite eines jeden Spannbackenpaares in Verschiebungsrichtung des Filmträgers relativ zum Geber des letzteren verstellbar sind und den Gebern ein Abnehmer zugeordnet ist, der mit den Führungen für den Filmträger verbunden und zusammen mit einem Istwertgeber für den Stellweg des Filmträgers an einer Messwertverarbeitungsanordnung angeschlossen ist. Bei einer solchen Ausbildung der Röntgenaufnahmeeinrichtung sind die Abstände der mit den Spannbacken gekuppelten Geber von dem mit dem Kassettenträger gekuppelten Geber proportional den an den zugehörigen Spannbacken abgegriffenen Kassettenmassen. Dies ist eine Grundvoraussetzung für eine einfache Messwertverarbeitung. Zugleich ist die Verstellung der Geber in Verschiebungsrichtung des Kassettenwagens eine Voraussetzung für weitere Ausgestaltungen der Erfindung.

In besonders vorteilhafter Weiterbildung der Erfindung kann der Abnehmer eine Lichtschranke sein, zwischen der flache, lichtundurchlässige Plättchen als Geber hindurchführbar sind. Hierdurch kann der Filmträger mit seinen Spannbacken ohne irgendwelche elektrischen Anschlüsse ausgeführt werden. Das hat zur Folge, dass der Filmträger ohne weiteres aus dem Röntgenuntersuchungsgerät herausgezogen bzw. herausgenommen werden kann. Dies kann u.a. der Fall sein, wenn durchleuchtet werden soll und die Verschiebbarkeit der Durchleuchtungseinrichtung in Tischlängsrichtung nicht durch einen am Fuss- oder Kopfende des Untersuchungstisches abgestellten Filmträger beeinträchtigt werden soll.

Eine besonders zuverlässige und zugleich einfache Konstruktion der Röntgenaufnahmeeinrichtung ergibt sich, wenn ein Geber in zweckmässiger Ausgestaltung der Erfindung mit einer Spannbacke über einen federbelasteten Seilzug verbunden ist. Dies führt zu einer platzsparenden und zugleich leicht zu reparierenden Konstruktion.

In besonders zweckmässiger Weiterbildung der Erfindung kann der Istwertgeber für den Stellweg des Filmträgers eine vorgegebene Zahl von Impulsen je zurückgelegter Wegstreckeneinheit erzeugen. Diese Bauweise hat nicht nur in Verbindung mit der linearen Verstellung der Geber im Filmträger eine lineare Messwertverarbeitung zur Folge, sondern führt auch zu einer relativ einfachen, weiterführenden Messwertverarbeitung mit relativ preiswerten digitalen Bauelementen.

In besonders vorteilhafter Ausgestaltung der Erfindung können die im Intervall zwischen den am Abnehmer vorbeigeführten Gebern des Filmträgers und einer Spannbacke der Messwertverarbeitungsanordnung zugeführten Impulse dort separat als Istwerteingabe des abgegriffenen Kassettenmasses gezählt werden. Die so gezählten Impulse sind ein direktes Mass für die entsprechende Breite bzw. Länge der Röntgenfilmkassette. Ihre Abnahme ist völlig unabhängig von der Geschwindigkeit, mit der der Filmträger mit den drei Gebern an den Abnehmern vorbeigeführt wird. Ausserdem kann so der Istwertgeber sowohl zur Positionierung des Filmträgers als auch zur Messung der Breite und Höhe der eingelegten Röntgenfilmkassette herangezogen werden, so dass die Bestimmung der Abmessungen der Röntgenfilmkassette und die entsprechende Verschiebung des Kassettenträgers von der Bereitschafts- in die Aufnahmestellung und wieder zurück von einem einzigen Impulsgeber und drei Messmarken bzw. Gebern vorgenommen werden kann. Dabei ist es gleich, ob ein Stellmotor gesteuert oder bei manueller Verstellung des Filmträgers dieser über Magnetbremsen in den Aufnahmepositionen arretiert wird.

Weitere Einzelheiten der Erfindung werden anhand eines in den Figuren dargestellten Ausführungsbeispieles erläutert. Es zeigen:

Fig. 1 ein Röntgenuntersuchungsgerät mit einem Untertischzielgerät in Seitenansicht, und

Fig. 2 eine schematische Darstellung des Stelltriebes für den Filmträger mit den Gebern und Abnehmern.

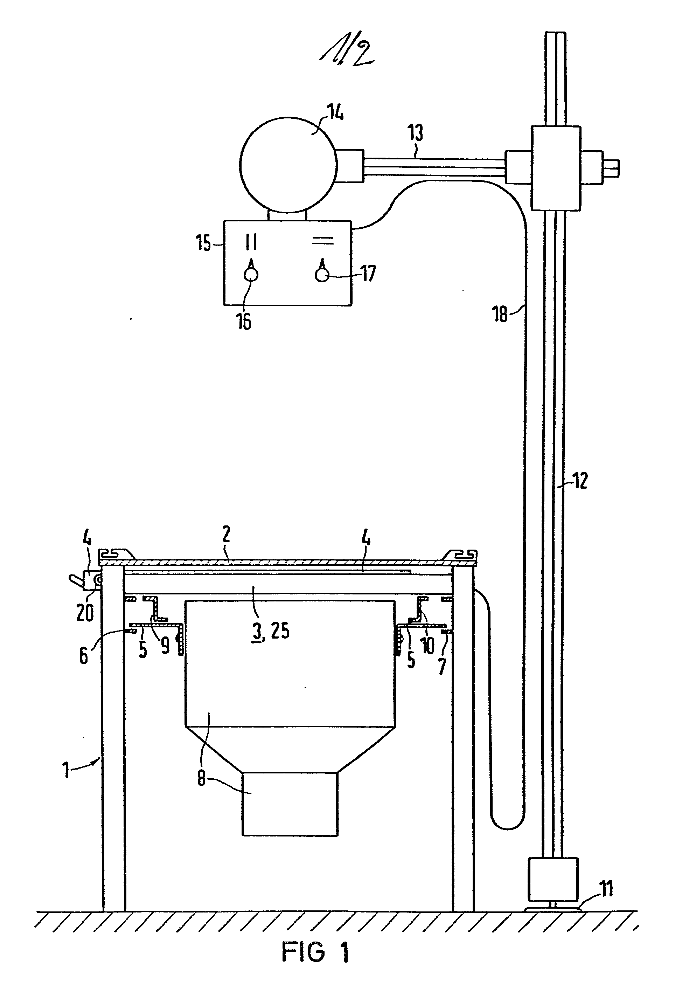

In der Fig. 1 erkennt man einen Untersuchungstisch 1 mit einer Patientenlagerungsplatte 2 und einem unter der Patientenlagerungsplatte in einen in Tischlängsrichtung verfahrbaren Längslaufwagen 3 einschiebbaren Filmträger 4, im Ausführungsbeispiel ein Kassettenblech 4. Unter dem Längslaufwagen des Untersuchungstisches ist eine in einem Halterungsblech 5 gelagerte, in Führungsschienen 6, 7 in Tischlängsrichtung verschiebbare Bildverstärker-Fernseheinrichtung 8 gelagert. Die Bildverstärker-Fernseheinrichtung 8 ist über zwei Kupplungsbleche 9, 10 mit dem Längslaufwagen 3 verbunden. Neben dem Untersuchungstisch 1 ist eine am Fussboden in einer Schiene 11 verfahrbare Stativsäule 12 zu erkennen, die über dem Untersuchungstisch an einem horizontalen Ausleger 13 eine Röntgenröhre 14 mit einer verstellbaren Primärstrahlenblende 15 trägt. Die Primärstrahlenblende ist mit Hilfe der Stellknöpfe 16, 17 manuell verstellbar. Diese Stellknöpfe sind ausserdem über fernsteuerbare Stelltriebe (nicht dargestellt) verstellbar. Zu diesem Zwecke ist die Primärstrahlenblende 15 über ein elektrisches Kabel 18 mit dem Untersuchungstisch 1 verbunden.

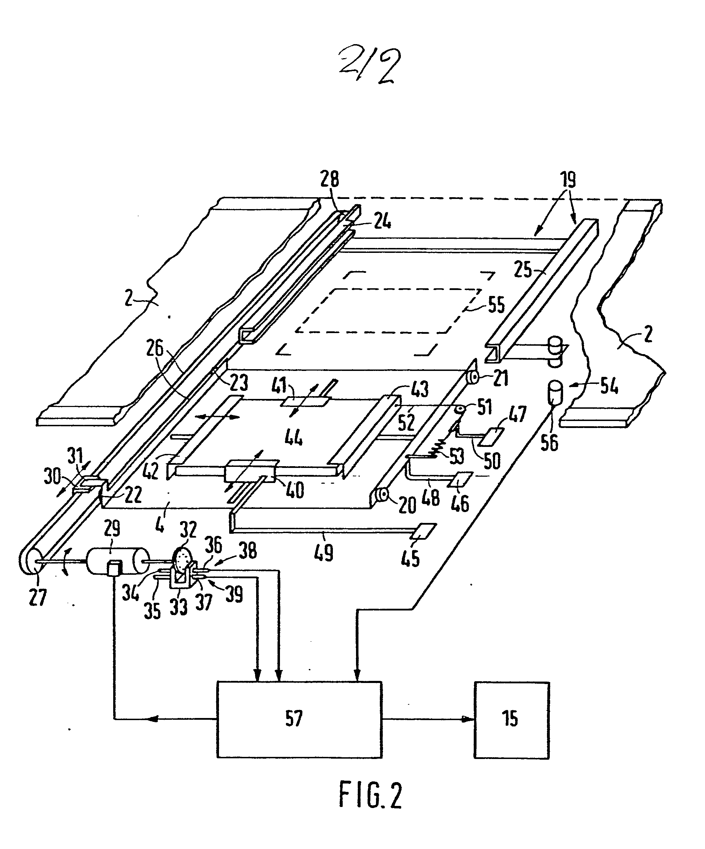

Die Fig. 2 zeigt in schematischer Darstellung einen Teil des Rahmens 19 des unterhalb der Patientenlagerungsplatte 2 in Längsrichtung des Untersuchungstisches 1 verschiebbaren Längslaufwagens 3. Das Kassettenblech 4 ist in der Fig. 2 aus dem Längslaufwagen herausgezogen dargestellt. Es trägt an seinen vier Ecken Kugellager 20, 21, 22, 23, mit denen es in den aus U-Schienen 24, 25 gefertigten, seitlichen Rahmenteilen des Längslaufwagens 3 quer zum Untersuchungstisch 1 einschiebbar ist. Längs des Kassettenblechs 4 ist ein endloses Transportband 26 zu erkennen. Das Transportband ist über zwei Walzen 27, 28 gespannt, von denen die eine von einem Motor 29 angetrieben ist. Die Walzen 27, 28 sind an den beiden Enden der in der Fig. 2 linken, der Übersichtlichkeit halber stark gekürzten U-Schiene 24 des Längslaufwagens 3 gelagert. Das Transportband 26 trägt ein Kupplungsstück 30, in das eine Zunge 31 des Kassettenblechs 4 einschiebbar ist.

Auf der Achse des Motors 29 ist eine Lochscheibe 32 verdrehungssicher befestigt. Diese Lochscheibe dreht sich mit ihren Löchern zwischen die auf gegenüberliegenden Seiten eines U-förmigen Tragkörpers 33 befestigten Lichtquellen 34, 35 und Fotozellen 36, 37 zweier Lichtschranken 38, 39. Der gegenseitige Abstand der beiden Lichtschranken 38, 39 beträgt ein ungeradzahliges Vielfaches des Lochabstandes auf der Lochscheibe 32.

Auf dem Kassettenblech 4 sind vier paarweise gegenläufig verstellbare Spannbacken 40, 41, 42, 43 zu erkennen. Sie werden über hier nicht weiter dargestellte, federbelastete Stelltriebe an die Kanten der eingesetzten Röntgenfilmkassette 44 angedrückt. Längs der in der Fig. 2 rechten Seite des Kassettenblechs 4, etwas gegenüber der Ebene des Kassettenblechs versetzt, befinden sich drei Reflexionsfolien 45, 46, 47. Die mittlere der drei Reflexionsfolien 46 ist über einen gewinkelten Halter 48 fest mit dem Kassettenblech 4 verbunden. Die in der Fig. 2 vordere Reflexionsfolie 45 ist über einen abgekröpften Halter 49 an einer Spannbacke 40 des in Stellrichtung des Kassettenblechs 4 verschiebbaren Spannbackenpaares befestigt. Die in der Fig. 2 hintere Reflexionsfolie 47 ist mit ihrem Halter 50 an einem um eine am Kassettenblech gelagerte Umlenkrolle 51 geführten Zugseil 52 befestigt. Dieses Zugseil ist mit seinem einen Ende mit einer Spannbacke 43 des quer zur Verschiebungsrichtung des Kassettenblechs 4 verschiebbaren Spannbackenpaares und mit seinem anderen Ende an einer am Kassettenblech eingehakten Zugfeder 53 befestigt. In den Stellweg dieser frei fluchtend zueinander gehaltenen Reflexionsfolien 45, 46, 47 ist eine am Längslaufwagen befestigte Lichtschranke 54 angeordnet. Im hinteren Teil der Fig. 2 ist zur Verdeutlichung das für eine Teilbelichtung der Röntgenfilmkassette 4 auszublendende Strahlenfeld 55 zwischen den U-Schienen 24, 25 gestrichelt eingezeichnet.

Sowohl die Fotozellen 36, 37 der beiden der Lochscheibe 32 zugeordneten Lichtschranken als auch die Fotozelle 56 der den Reflexionsfolien 45, 46, 47 zugeordneten Lichtschranke 54 sind an eine Messwertverarbeitungsanordnung 57 angeschlossen. Diese wiederum ist mit dem Motor 29 für die Verschiebung des Kassettenblechs 4 und mit der Primärstrahlenblende 15 verbunden.

Solange das Kassettenblech aus dem rahmenförmig ausgebildeten Längslaufwagen 3 herausgenommen ist, ist letzterer schattenfrei durchstrahlbar. Das bedeutet, dass er als Rahmen 19 mit der darunter befindlichen Bildverstärker-Fernseheinrichtung 8 verbunden sein kann. In diesem Fall behindert er auch nicht die Verschiebbarkeit der Bildverstärker-Fernseheinrichtung in Längsrichtung des Untersuchungstisches 1. Soll eine Röntgenaufnahme mit Hilfe einer Röntgenfilmkassette 44 angefertigt werden, so kann das Kassettenblech 4 in die U-Schienen 24, 25 des Längslaufwagens 3 eingeschoben und dabei mit seiner Zunge 31 an das Kupplungsstück 30 des Transportbandes 26 angekuppelt werden. Nunmehr kann eine Röntgenfilmkassette 44 geeigneter Grösse zwischen die Spannbacken 40, 41, 42, 43 eingesetzt werden. Nach dem Einsetzen der Röntgenfilmkassette 44 legen sich die Spannbacken 40, 41, 42, 43 in bekannter Weise an die Stirnkanten der Röntgenfilmkassette an und zentrieren diese zur Mitte des Kassettenblechs 4. Nunmehr entspricht der Abstand der mit den beiden verstellbaren Spannbacken 40, 43 verbundenen Reflexionsfolien 45, 47 zu der starr am Kassettenblech 4 befestigten Reflexionsfolie 46 der Höhe bzw. der Breite der Röntgenfilmkassette.

Erscheint während der Untersuchung mit der Bildverstärker-Fernseheinrichtung 8 ein Befund der festgehalten werden soll, so kann der Motor 29 für den Transport des Kassettenblechs 4 in hier nicht weiter dargestellter Weise eingeschaltet werden. Durch ihn wird das Transportband 26 mit dem Kupplungsstück 30 und das über die eingeschobene Zunge 31 mit dem Transportband gekuppelte Kassettenblech in die Aufnahmeposition verfahren. Dabei zählen die beiden, der runden Lochscheibe 32 zugeordneten Lichtschranken 38, 39 die an ihnen vorbeigeführten Löcher der Lochscheibe 32. Ihre Zahl ist – wie das im einzelnen bereits in der DE-A-2 440 146 beschrieben ist – ein Mass für den Betrag und für die Richtung des zurückgelegten Stellwegs. Beim Verfahren des Kassettenblechs passieren die seitlich aus dem Kassettenblech herausgeführten Reflexionsfolien 45, 46, 47 die Lichtschranke 54. Dabei erzeugt die Fotozelle 56 dieser Lichtschranke je einen Impuls, der an die Messwertverarbeitungsanordnung 57 weitergeleitet wird. Die in dem Zeitintervall zwischen dem Passieren der mit jeweils einer Spannbacke 40, 43 verbundenen Reflexionsfolie 45, 47 und der mit dem Kassettenblech 4 fest verbundenen Reflexionsfolie 46 eingehenden, über die Lochscheibe 32 erzeugten Impulse werden in jeweils einem besonderen Zähler der Messwertverarbeitungsanordnung 57 gezählt. Sie sind ein Mass für die Länge bzw. für die Breite der auf dem Kassettenblech 4 eingespannten Röntgenfilmkassette 44 und können von der Messwertverarbeitungsanordnung zur Steuerung der Einfahrt des Kassettenblechs in die Aufnahmeposition herangezogen werden.

Weil die jeweils einem Impuls entsprechende Wegstrecke in allen drei Fällen exakt gleich ist, lassen sich die 1:2 untersetzten, für die Kassettenabmessungen gezählten Impulse, direkt zu den vorgegebenen, dem Normalstellweg des Kassettenblechs für ungeteilte Aufnahmen entsprechenden Impulsen ab- bzw. zuzählen, um den Stellmotor in den beiden so errechneten Aufnahmepositionen bei zweifach unterteilter Aufnahme abzuschalten. Mit dem separat gespeicherten, den Kassettenabmessungen entsprechenden Impulsen lässt sich eine Nachlaufsteuerung für die Einstellung der Primärstrahlenblende 15 speisen. In gleicher Weise, wie im Ausführungsbeispiel die Verstellung des Kassettenblechs 4 quer zum Untersuchungstisch 1 dargestellt ist, lässt sich auch der Längslaufwagen 3 oder ein Teil desselben mit den U-Schienen aufgrund der abgegriffenen Kassettenmasse zur Filmunterteilung in Tischlängsrichtung verstellen. Es ist ein besonderer Vorzug dieser Bauweise, dass man mit nur einem Motor 29, drei Lichtschranken 38, 39, 54 und drei Reflexionsfolien 45, 46, 47 auskommt, um eine vollautomatische Abtastung der Kassettenmasse und Steuerung des Vorschubs des Kassettenblechs in der Aufnahmestellung zu erreichen. Dabei lassen sich anstelle der Reflexionsfolien ebensogut auch Permanentmagnete und anstelle der Lichtschranken Reed-Kontakte oder Spulen verwenden.

## Patentansprüche

1. Röntgenaufnahmeeinrichtung mit einem in Führungen von einer Bereitschaftsstellung in mindestens eine Aufnahmestellung und wieder zurück verfahrbaren Filmträger mit verschiebbaren Spannbacken für die Halterung von Röntgenfilmkassetten unterschiedlicher Formates, dadurch gekennzeichnet, dass der Filmträger (4) und zumindest ein Teil der Spannbacken (40, 41, 42, 43) mit Gebern (45, 46, 47) gekuppelt sind, wobei die Geber (45, 47) der Spannbacken (40, 43) in Abhängigkeit von der Spannweite eines jeden Spannbackenpaares in Verschiebungsrichtung des Filmträgers (4) relativ zum Geber (46) des letzteren verstellbar sind und den Gebern (45 bis 47) ein Abnehmer (54) zugeordnet ist, der mit den Führungen für den Filmträger (4) verbunden und zusammen mit einem Istwertgeber (32, 38, 39) für den Stellweg des Filmträgers an einer Messwertverarbeitungsanordnung (57) angeschlossen ist.

2. Röntgenaufnahmeeinrichtung nach Anspruch 1, dadurch gekennzeichnet, dass der Abnehmer eine Lichtschranke (54) ist, zwischen der flache, lichtundurchlässige Plättchen (45, 46, 47) als Geber hindurchführbar sind.

3. Röntgenaufnahmeeinrichtung nach Anspruch 1, dadurch gekennzeichnet, dass ein Geber (47) mit einer Spannbacke (43) über einen federbelasteten Seilzug (52) verbunden ist.

4. Röntgenaufnahmeeinrichtung nach Anspruch 1, dadurch gekennzeichnet, dass der Istwertgeber (32, 38, 39) für den Stellweg des Filmträgers (4) eine vorgegebene Zahl von Impulsen je zurückgelegter Wegstreckeneinheit erzeugt.

5. Röntgenaufnahmeeinrichtung nach Anspruch 4, dadurch gekennzeichnet, dass die im Intervall zwischen den am Abnehmer (54) vorbeigeführten Gebern (45, 46, 47) des Filmträgers (4) und einer Spannbacke (40, 43) der Messwertverarbeitungsanordnung (57) zugeführten Impulse dort separat als Istwerteingabe des abgegriffenen Kassettenmasses gezählt werden.

6. Röntgenaufnahmeeinrichtung nach Anspruch 4, dadurch gekennzeichnet, dass die Impulse durch eine mit einem Transportband (26) für den Filmträger (4) gekuppelte, sich im Lichtstrahl mindestens einer Lichtschranke (38, 39) drehenden Lochscheibe (32) erzeugt werden.

7. Röntgenaufnahmeeinrichtung nach Anspruch 6, dadurch gekennzeichnet, dass die Lochscheibe (32) mit der Antriebswelle eines Motors (29) zum Antrieb des Transportbandes (26) des Filmträgers (4) verbunden ist.

8. Röntgenaufnahmeeinrichtung nach Anspruch 6, dadurch gekennzeichnet, dass der Lochscheibe (32) zur drehsinnabhängigen Messung zwei um ungeradzahlige Vielfache des Lochabstandes gegeneinander versetzt angeordnete Lichtschranken (38, 39) zugeordnet sind.

9. Röntgenaufnahmeeinrichtung nach Anspruch 1, dadurch gekennzeichnet, dass jeweils nur eine Spannbacke (40, 43) eines Spannbackenpaares mit einem Geber (45, 47) verbunden ist und die jeweils gegenüberliegende Spannbacke (42, 41) symmetrisch gegenläufig zur erstgenannten Spannbacke bewegt wird.

10. Röntgenaufnahmeeinrichtung nach Anspruch 1, dadurch gekennzeichnet, dass jeweils nur eine Spannbacke eines Spannbackenpaares mit einem Geber verbunden ist, die jeweils gegenüberliegende Spannbacke dagegen als Festanschlag ausgebildet ist.

11. Röntgenaufnahmeeinrichtung nach Anspruch 1, dadurch gekennzeichnet, dass als Abnehmer ein Reed-Kontakt und als Geber Permanentmagnete verwendet sind.

## Revendications

1. Dispositif de prise de vues radiographiques comportant un porte-film déplaçable dans des guides depuis une position de disponibilité dans une position de prise de vues et inversement et possédant des mâchoires de serrage déplaçables servant au maintien de cassettes à film radiographique possédant des formats différents, caractérisé par le fait que le porte-film (4) et au moins une partie des mâchoires de serrage (40, 41, 42, 43) sont accouplées à des capteurs (45, 46, 47), les capteurs (45, 47) des mâchoires de serrage (40, 43) étant réglables, en fonction de la course de serrage de chaque couple de mâchoires de serrage, suivant la direction de déplacement du porte-film (4) par rapport au capteur (46) de ce dernier, tandis qu'aux capteurs (45 à 47) est associé un détecteur (54) qui est relié aux guides du porte-film (4) et est raccordé, ainsi qu'un générateur de valeurs réelles (32, 38, 39) pour le trajet de réglage du porte-film, à un dispositif (57) de traitement des valeurs de mesure.

2. Appareil de prise de vues radiographiques suivant la revendication 1, caractérisé par le fait que le détecteur est un relais photoélectrique (54) à travers lequel des plaquettes ou plaques, opaques à la lumière (45, 46, 47) servant de capteurs peuvent être introduites.

3. Appareil de prise de vues radiographiques suivant la revendication 1, caractérisé en ce qu'un capteur (47) est relié à une mâchoire de serrage (43) par l'intermédiaire d'un câble de transmission (52) chargé par un ressort.

4. Appareil de prises de vue radiographiques suivant la revendication 1, caractérisé par le fait que le générateur de valeurs (32, 38, 39) indiquant le trajet de réglage du porte-film (4) produit un nombre prédéterminé d'impulsions pour chaque unité parcourue dudit trajet.

5. Appareil de prise de vues radiographiques suivant la revendication 4, caractérisé par le fait que les impulsions, qui sont envoyées dans l'intervalle compris entre les capteurs (45, 46, 47) passant devant le détecteur (54), du porte-film (4) et une mâchoire de serrage (40, 43) du dispositif (57) de traitement des valeurs de mesure, sont comptées séparément sous la forme d'une introduction de la valeur réelle de la dimension détectée de la cassette.

6. Appareil de prise de vues radiographiques suivant la revendication 4, caractérisé par le fait que les impulsions sont produites par un disque perforé (32) accouplé à une bande de transport (26) prévue pour le porte-film (4), et tournant dans le faisceau lumineux d'au moins un relais photoélectrique (38, 39).

7. Appareil de prise de vues radiographiques suivant la revendication 6, caractérisé par le fait que le disque perforé (32) est relié à l'arbre d'entraînement d'un moteur (29) servant à entraîner la bande de transport (26) pour le porte-film (34).

8. Appareil de prise de vues radiographiques suivant la revendication 6, caractérisé par le fait que deux relais photélectriques (38, 39) disposés en étant décalés d'un multiple impair de la distance réciproque des trous du disque (32) sont associés à ce dernier pour effectuer une mesure qui est fonction du sens de rotation.

9. Appareil de prise de vues radiographiques suivant la revendication 1, caractérisé par le fait que seule une mâchoire de serrage (40, 43) d'un couple de mâchoires de serrage est reliée à un capteur (45, 47) et que la mâchoire de serrage (42, 41) disposée en vis-à-vis est déplacée d'une manière symétrique et en sens inverse de la mâchoire de serrage indiquée en premier lieu.

10. Appareil de prise de vues radiographiques suivant la revendication 1, caractérisé par le fait que seule une mâchoire de serrage d'un couple de mâchoire de serrage est reliée à un capteur, tandis que l'autre mâchoire de serrage disposée en vis-à-vis est réalisée sous la forme d'une butée fixe.

11. Appareil de prise de vues radiographiques suivant la revendication 1, caractérisé par le fait qu'on utilise un contact à lame comme détecteur et des aimants permanents comme capteurs.

## Claims

1. An X-ray exposure device with a film carrier which can be moved in guides from a parked position into at least one exposure position and vice versa, with displaceable clamping jaws which serve to hold X-ray film cassettes of different format, characterised in that the film carrier (4) and at least a part of the clamping jaws (40, 41, 42, 43) are coupled to markers (45, 46, 47), where the markers (45, 47) of the clamping jaws (40, 43) can be adjusted, in dependence upon the clamping width of each pair of clamping jaws, in the direction of movement of the film carrier (4) relative to the marker (46) of the latter, and the markers (45 to 47) are assigned a detector (54) which is connected to the guides for the film carrier (4) and, together with an actual-value generator (32, 38, 39) for the control path of the film carrier, is connected to a measured-value processing arrangement (57).

2. An X-ray exposure device as claimed in Claim 1, characterised in that the detector is an optical sensor (54) between which markers in the form of flat, light-impermeable discs (45, 46, 47) can be passed.

3. An X-ray exposure device as claimed in Claim 1, characterised in that one marker (47) is connected to a clamping jaw (43) via a spring-loaded cable line (52).

4. An X-ray exposure device as claimed in Claim 1, characterised in that the actual-value generator (32, 38, 39) for the control path of the film carrier (4) generates a predetermined number of pulses in respect of each distance unit which is passed over.

5. An X-ray exposure device as claimed in Claim 4, characterised in that the pulses which are supplied in the interval between the markers (45, 46, 47) of the film carrier (4) and of one of the clamping jaws (40, 43) moving past the detector (54) of the measured-value processing arrangement (57) are counted separately at this point as actual-value input of the read-off cassette measurements.

6. An X-ray exposure device as claimed in Claim 4, characterised in that the pulses are generated by a perforated disc (32) coupled to a transport belt (36) for the film carrier (4) to rotate in the light beam of at least one optical sensor (38, 39).

7. An X-ray exposure device as claimed in Claim 6, characterised in that the perforated disc (32) is connected to the drive shaft of a motor (29) which serves to drive the transport belt (26) of the film carrier (4).

8. An X-ray exposure device as claimed in Claim 6, characterised in that for measurement that is dependent upon rotation-direction the perforated disc (32) is assigned two optical sensors (38, 39) arranged offset relative to one another by odd-numbered multiples of the interval between the perforations.

9. An X-ray exposure device as claimed in Claim 1, characterised in that only one clamping jaw (40, 43) of each pair of clamping jaws is connected to a marker (45, 47) and the particular opposite clamping jaw (42, 41) is moved in symmetrical opposition to the first mentioned clamping jaw.

10. An X-ray exposure device as claimed in Claim 1, characterised in that onyl one clamping jaw of each pair of clamping jaws is connected to a marker, whereas the particular opposite clamping jaw consists of a fixed stop means.

11. An X-ray exposure device as claimed in Claim 1, characterised in that a reed relay contact is used as a detector collector and permanent magnets are used as markers.

FIG 1

2/2

FIG.2